# EUROPÄISCHE PATENTSCHRIFT

(11) **EP 3 615 120 B1**
(45) Veröffentlichungstag und Bekanntmachung des Hinweises auf die Patenterteilung: **23.10.2024**
(21) Anmeldenummer: 18709914.8
(22) Anmeldetag: 16.02.2018
(51) Int. Cl.: A61M 16/20, A61M 16/08, A61M 16/10

(54) **PATIENTENVENTIL ZUR BEATMUNG EINES PATIENTEN MIT EINEM BEATMUNGSGERÄT**
PATIENT VALVE FOR VENTILATING A PATIENT WITH A VENTILATOR
VALVE POUR PATIENT POUR METTRE EN OEUVRE UNE RESPIRATION ARTIFICIELLE SUR UN PATIENT AU MOYEN D'UN VENTILATEUR MÉCANIQUE

(30) Priorität: 27.04.2017 DE 102017004137; 10.11.2017 DE 102017010485
(43) Veröffentlichungstag der Anmeldung: 04.03.2020
(73) Patentinhaber: WEINMANN Emergency Medical Technology GmbH + Co. KG, 22525 Hamburg (DE)
(72) Erfinder: NEUHAUS, Christian, 25451 Quickborn (DE); PULLA, Matthias, 22459 Hamburg (DE); KREUZER, Johannes, 21079 Hamburg (DE); EL DIWANY, Samir, 22850 Norderstedt (DE)
(74) Vertreter: Klickow & Wetzel PartGmbB
(86) Internationale Anmeldenummer: PCT/DE2018/000041
(87) Internationale Veröffentlichungsnummer: WO 2018/196895

(56) Entgegenhaltungen:
- EP-A1- 2 359 889
- WO-A1-00/45883
- WO-A1-01/66175
- WO-A1-2009/089807
- WO-A1-2010/061173
- DE-A1- 10 035 938
- DE-A1- 102008 026 321
- DE-U1- 202015 100 434
- DE-U1- 202015 100 434
- GB-A- 1 602 925
- US-A- 2 834 339
- US-A- 3 063 620
- US-A1- 2008 092 898
- US-A1- 2015 231 028

## Beschreibung

Die Erfindung betrifft ein Patientenventil zur Beatmung eines Patienten mit einem Beatmungsgerät aufweisend ein erstes Ventilelement mit wenigstens einem Anschluss.

In der modernen Medizin werden in einer Vielzahl unterschiedlicher Situationen Medikamente verschiedenster Art eingesetzt, um akute und chronische Symptome verursacht durch Krankheiten, schädliche äußere Einwirkungen und unfallbedingte Schäden zu lindern oder zu beseitigen. Auch in Notfallsituationen werden Medikamente eingesetzt, um beispielsweise ein drohendes Versagen der Vitalfunktionen eines Unfallopfers oder ein krankheitsbedingtes Kollabieren von Personen zu verhindern.

In Notfallsituationen, insbesondere bei ambulanten Erstversorgungen von Patienten, ist es häufig erforderlich, dass Sauerstoff über eine Notfallbeatmung verabreicht wird. Um diese mit Sauerstoff angereicherte Atemluft dem Patienten vor Ort zuführen zu können, werden mobile und ggfs. auch tragbare, in der Regel pneumatisch arbeitende Beatmungsgeräte beispielsweise in Rettungsfahrzeugen oder Notarztwagen mitgeführt, um an einem Notfallort verfügbar zu sein.

Eine Variante der Beatmungstechnik verzichtet auf die Anreicherung von Sauerstoff in die Atemluft. Die Atmung eines Patienten wird in diesen Fällen durch die Atemluftzuführung mit einem gegenüber Atmosphäre leichtem Überdruck unterstützt. Beide Varianten - sowohl mit als auch ohne Sauerstoffanreicherung - werden als nicht-invasive Beatmung mittels Überdruck bezeichnet. Ebenfalls ist es möglich, invasiv zu Beatmen beispielsweise durch Intubieren.

Die Vorrichtungen für die nicht-invasive Beatmung mittels Überdruck sind primär gebildet durch die eigentlichen Beatmungsgeräte sowie den Verbindungssystemen zwischen Beatmungsgerät und dem zu beatmenden Patienten. Das Verbindungssystem verfügt wenigstens über eine Atemluftführung meist gebildet aus einem Schlauch sowie einer Kontaktschnittstelle beziehungsweise einem Interface in Form einer Atemmaske (Nase-, Nase-Mund-, Gesichts-) oder einem Atemhelm. Ergänzt werden kann das Verbindungssystem durch ein sogenanntes Patientenventil, das je nach Anforderungen eine oder mehrere Funktionen in sich vereinigt. Insbesondere kann das Patientenventil als Wegeventil ausgebildet sein.

In der Beatmungstechnik und insbesondere in der Notfallbeatmung ist es wichtig, möglichst leichte, schlanke und totraumreduzierte Verbindungssysteme und/oder Kontaktschnittstellen zum Atemweg des Patienten zu haben.

Unter Totraum (oder auch Atemraum) versteht man im engeren Sinn bei Atemmasken den Zwischenraum zwischen Maske und Gesicht des Patienten. Das bedeutet, dass der Totraum der Innenmaske begrenzt beziehungsweise minimiert wird. Dort hinein atmet der Atemmaskenträger aus. Der Totraum muss so klein wie möglich sein, da sonst dort ein großer Teil der kohlendioxidhaltigen Ausatemluft verbleibt und mit all ihren schädlichen Folgen wieder eingeatmet wird.

Der Totraum kann durch das der Atemmaske direkt nachgeschaltete Patientenventil negativ, das heißt mit vergrößernder Wirkung, beeinflusst werden. Je nach realisierter Ausführung und konstruktivem Aufbau kann in diesem Zusammenhang das vermeidbare atemluftführende Volumina des Verbindungssystems den Totraum in unerwünschter Weise vergrößern.

Möglichst leichte, schlanke und totraumreduzierte Verbindungssysteme und/ oder Kontaktschnittstellen zum Atemweg des Patienten vermindern weiterhin das Risiko einer unerwünschten und möglicherweise lebensgefährlichen Extubation, das heißt die Entfernung des Verbindungssystems beziehungsweise Kontaktverlust des Interfaces zum Patienten.

Weiterhin unterstützen leichte, schlanke und totraumreduzierte Verbindungssysteme und/oder Kontaktschnittstellen die Beatmung von kleineren Patienten, insbesondere Kindern und erleichtert dem Anwender die Arbeit.

Zusätzlich sollen Verbindungssysteme, Interfaces und Patientenventile für verschiedene Beatmungsgeräte mit unterschiedlichen Anschlüssen, beispielsweise variierenden Nennweiten oder Zusatzfunktionen, eingesetzt werden können. Wichtig ist unter anderem, dass die anzuschließenden Komponenten wie beispielsweise Schläuche, Verkabelungen, Messgeräte, Steuergeräte usw. in korrekter Weise und ohne Verwechselungen angeschlossen werden. Auch ist ein sicheres Anbringen der nötigen Schläuche zu gewährleisten.

In der WO 2009/089807 A1 wird bereits ein Patientenventil mit einem ersten Ventilelement und einem Anschluss beschrieben. Der Anschluss ist relativ zu einem Umfang eines Oberteiles des Patientenventils in einer im wesentlichen horizontalen Ebene abgewinkelt angeordnet.

Aus der GB 1 602 925 A ist ein weiteres Patientenventil bekannt, dass ein Anschluss für einen Schlauch aufweist.

In der DE 100 35 938 A1 wird ein weiteres Patientenventil beschrieben, dass ein Anschlussstutzen für einen Schlauch aufweist. Ebenfalls wird eine derartige Konstruktion in der WO 01/661175 A1 beschrieben.

Aus der DE 20 2015 100 434 U1 ist bereits eine Atemmaske mit einem Anschlussstutzen zur Zuführung von Sauerstoff bekannt. Gemäß einem ersten Ausführungsbeispiel ist der Anschlussstutzen seitlich und senkrecht zu einer Atemgaszuführung angeordnet, gemäß einem zweiten Ausführungsbeispiel ist der Stutzen unmittelbar an der Atemmaske und parallel zur Atemgaszuführung angeordnet.

Die DE 10 2008 026321 A1 offenbart ein Patientenventil gemäß dem Oberbegriff des Anspruchs 1.

Es ist Aufgabe der Erfindung, die bestehenden Patientenventile als Bestandteile der Verbindungssysteme zwischen Beatmungsgerät und dem zu beatmenden Patienten weiterzuentwickeln und die zuvor genannten Nachteile wenigstens teilweise zu reduzieren.

Die Aufgabe wird erfindungsgemäß durch die Merkmale von Patentanspruch 1 gelöst. Die Unteransprüche definieren Ausführungsbeispiele der Erfindung.

Die folgende allgemeine Beschreibung offenbart Ausführungsbeispiele, die nicht unbedingt erfindungsgemäß sind, aber Merkmale enthalten, die in erfindungsgemäßen Ausführungsbeispielen angewendet werden können.

Die Lehre dieser Offenbarung schlägt ein Patientenventil als Komponente des Verbindungssystems vor, das die Modularität des Verbindungssystems unterstützt und auf diese Weise für unterschiedliche Beatmungsgeräte und/oder Patienten einsetzbar ist. Durch eine kompakte Bauform wird ein möglichst kleiner Totraum realisiert und ein zum Beatmungsschlauch hin gerichtetes Wegführen von Anschlüssen erlaubt die leichte und schlanke Bauform. Die Anschlüsse sind verwechselungs- und, oder verstecksicher ausgeführt und die einzelnen Verbindungen müssen nicht verkleben werden. Ein optionaler Ventiladapter ist austauschbar realisiert.

Das neuartige Patientenventil ist wenigstens einteilig und vorzugsweise als Ausatemventil für Beatmungsgeräte, Beatmungsbeutel und/oder Notfallbeatmungsgeräte konzipiert. Der wenigstens eine Anschluss des Patientenventils ist abgewinkelt ausgeführt, das heißt in einem Winkel abweichend von der Lotrechten zur Patientenventil-Mittenachse.

Durch die beschriebene Konstruktion kann das Patientenventil kürzer ausgebildet werden und unterstützt auf diese Weise die Totraumreduzierung. Diese Offenbarung erkennt, dass ein Winkelbereich von ca. 25 Grad bis ca. 75 Grad und insbesondere ein Winkelbereich von ca. 30 Grad bis ca. 50 Grad zwischen Anschluss- und Patientenventilmittenachse die Verkürzung der Konstruktion unterstützt.

Basierend auf einem weiteren Gedanken kann die Kompaktheit des Patientenventils eine Totraumreduzierung unterstützen: Sind zwei oder mehrere Anschlüsse vorgesehen, können diese alternativ oder additiv zu der Abwinkelung von einer in Längsrichtung fluchtenden Anordnung abweichend positioniert werden. Dies kann beispielsweise durch eine Positionierung in Umfangsrichtung auf in etwa gleicher Höhe und unter einem Versatzwinkel zueinander erfolgen.

Einem Verwechselungs- oder Versteckrisiko, das heißt einer fehlerhaften Zuordnung von Schläuchen, Verkabelungen, Messgeräten, Steuergeräten usw. zum jeweiligen Anschluss wird durch geeignete Geometrien und/oder Größendimensionen entgegengewirkt.

Wird das Patientenventil zweiteilig ausgeführt, ist vorgesehen, den Anschluss zwischen den Teilen des Patientenventils konisch auszubilden und Verwechselungs- bzw. verstecksicher zu gängigen Beatmungskonen zu gestalten. Übliche Beatmungskonendimensionen weisen 22mm und/oder 15 mm auf. Der Anschluss zwischen den Teilen des Patientenventils ist immer gleich, hierdurch können beliebige Kombinationen mit funktionell unterschiedlichen Komponenten erstellt werden.

Um eine Modularität des Verbindungssystems zusätzlich oder alternativ zu unterstützen und die Kompatibilität zu verschiedenen Interfaces zu gewährleisten, erfolgt der Anschluss des Atemweges des Patienten über Normkonen aus der Beatmungstechnik, das heißt 15mm oder 22mm Innenmaß.

In einer beispielhaften Ausführungsform des Patientenventils ist eine Variante mit integriertem Druckabgriff zur Beatmungssteuerung vorgesehen, welcher einen Anschluss der Ausatemmembran an den Beatmungsschlauch über eine definierte Drossel erlaubt.

Eine weitere Variante des Patientenventils kann wenigstens einen Anschluss zur Einspeisung von Sauerstoff in den Beatmungsschlauch aufweisen, beispielsweise um diesen zu puffern.

Das Patientenventil kann optional wenigstens einen Anschluss für eine Druckmessung aufweisen, beispielsweise zur Messung des Atemwegsdrucks des Patienten.

Alternativ oder additiv kann das Patientenventil wenigstens einen Anschluss zur Absaugung von (Aus)Atemgasen zur Gasanalyse aufweisen. Gleiches gilt für Steuerleitungen verschiedener Art.

Jeder der Anschlüsse kann mit oder ohne Rückschlagfunktion, beispielsweise realisiert über eine Membran oder ein Wegeventil, ausgebildet sein.

Der Abgang des Patientenventils in Richtung des Beatmungsschlauches kann auch als Blindstopfen ausgeführt werden, sodass die Ausatmungsluft in die Umgebung geleitet wird. Die Zuführung der Einatemluft erfolgt typischer Weise nicht durch das Ventil hindurch. Das Ventil dient bevorzugt nur zur Ableitung der Ausatemluft.

Ebenfalls kann optional vorgesehen sein, dass das Patientenventil für die Einweg-Verwendung konzipiert ist.

Durch eine optionale Rückschlagmembran in dem atemluftführenden Kanal des Patientenventils kann eine weitere Variante realisiert sein. Die Rückschlagmembran, die funktional als Wegeventil fungiert, sorgt dafür dass ein Rückatmen in den Beatmungsschlauch verhindert ist.

In den Zeichnungen sind Ausführungsbeispiele dargestellt. Es zeigen:
- Fig. 1: eine erste mögliche Ausgestaltung des erfindungsgemäßen Patientenventils (1) mit Druckregelanschluss (50, 51) in Verbindung mit integriertem Druckabgriff (50, 52) und Sauerstoffeinspeisung (50, 55),
- Fig. 2: eine weitere mögliche, nicht erfindungsgemäße, Variante des Patientenventils (1) mit Druckregelanschluss (50, 51) und ohne Sauerstoffeinspeisung (50, 55) sowie ohne integriertem Druckabgriff (50, 52) und
- Fig. 3: die Schnittdarstellung des erfindungsgemäßen Patientenventils (1) aus Figur 1 mit Druckregelanschluss (50, 51) in Verbindung mit integriertem Druckabgriff (50, 52) und Sauerstoffeinspeisung (50, 55).

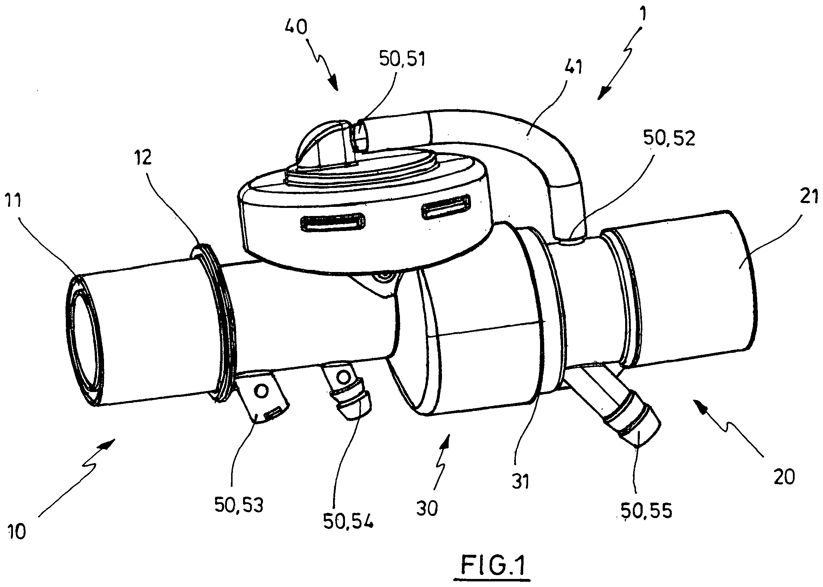

Figur 1 bildet in einer dreidimensionalen Darstellung eine erste mögliche Ausgestaltung des erfindungsgemäßen Patientenventils (1) in einer mehrteiligen Variante ab. Das Patientenventil (1) ist hier als Ausatemventil ausgeführt und gebildet durch ein erstes Ventilelement (10) und ein zweites Ventilelement (20) in Kombination mit einem Drucksteuerelement (40). Das Drucksteuerelement (40) ist integrativer Bestandteil des ersten Ventilelementes (10), sodass das exemplarische Patientenventil (1) in dieser Variante zweiteilig und mit einer Verbindungsstelle (30) ausgeführt ist. Möglich sind auch Ausgestaltungen in einteiliger (nicht erfindungsgemäß) und/oder wenigstens dreiteiliger Realisierung.

Das Drucksteuerelement (40) ist hier ausgeführt mit einem Druckregelanschluss (50, 51), der über eine Steuerleitung (41) in Wirkverbindung steht mit einem - in dieser Variante vorgesehenen - integriertem Druckabgriff (50, 52) des zweiten Ventilelementes (20).

In diesem beispielhaften Patientenventil (1) kommt eine Variante des zweiten Ventilelementes (20) zur Anwendung, das über eine Sauerstoffeinspeisung (50, 55) verfügt. Der Anschluss eines Beatmungsschlauches und/oder eines Blindstopfens wird durch das vorgesehene Anschlussstück (21) unterstützt. Die in Figur 1 gezeigte einstückige Kombination aus dem ersten Ventilelement (10) mit der Drucksteuerelement (40) wird über eine Verbindungsstelle (30) mit dem zweiten Ventilelement (20) gekoppelt. Die Verbindungsstelle (30) mit ihrer Trennebene (31) ist derart ausgestaltet, dass modulartig verschiedene zweite Ventilelemente (20) oder ein Blindstopfen anstelle des zweiten Ventilelementes (20) koppelbar sind.

Das einstückige Ausführungsbeispiel des ersten Ventilelementes (10) mit Drucksteuerelement (40) verfügt über wenigstens einen Anschluss (50) in abgewinkelter Form, das heißt in einem Winkel abweichend von der Lotrechten zur Patientenventil-Mittenachse. Durch die erfindungsgemäße Konstruktion kann das Patientenventil kürzer ausgebildet werden und unterstützt auf diese

Weise die Totraumreduzierung. Es sind Anschlüsse (50) vorgesehen als Messanschluss CO2 (53) und als Atemwegdruck-Messanschluss (54).

Das erste Ventilelement (10) verfügt in der Regel über einen Atemweg- Anschlussbereich (11), der häufig konisch ausgeführt ist, sowie über einen Kragen (12), der als axiale Anlageschulter für ein Interface, einen Schlauch oder eine Muffe dient.

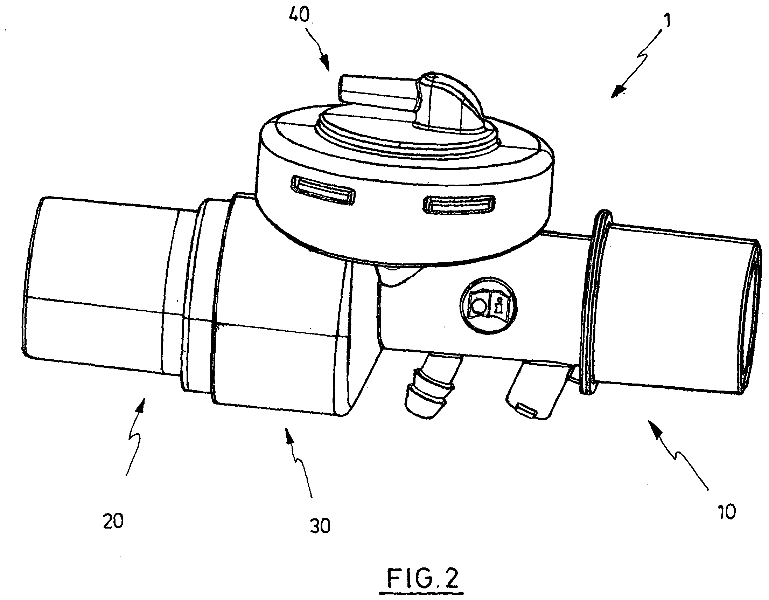

Figur 2 zeigt in perspektivischer Ansicht eine weitere mögliche, nicht erfindungsgemäße, Variante des Patientenventils (1) mit Druckregelanschluss (50, 51) und ohne Sauerstoffeinspeisung sowie ohne integriertem Druckabgriff. Diese Variante verdeutlicht die modulare Möglichkeit unterschiedlich gestalteter erster und/oder zweiter Ventilelemente (10, 20) durch die auf verschiedene Varianten abgestimmte Verbindungsstelle (30).

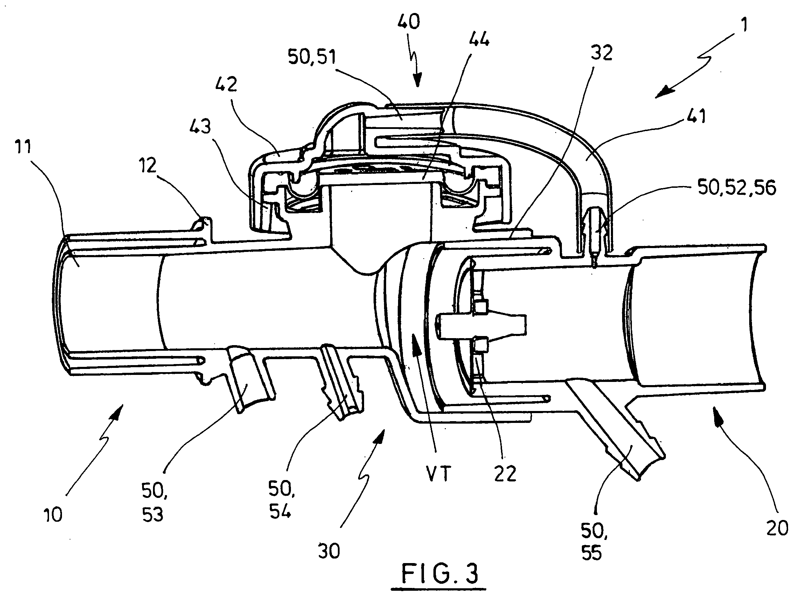

Figur 3 umfasst die räumliche Schnittdarstellung des erfindungsgemäßen Patientenventils (1) aus Figur 1 mit Druckregelanschluss (50, 51) in Verbindung mit integriertem Druckabgriff (50, 52) und Sauerstoffeinspeisung (50, 55). Diese Variante des Patientenventils (1) ist als Ausatemventil konzipiert und verfügt über ein Drucksteuerelement (40), das mit einer Steuermembrane (44) ausgestattet ist. Atemwegsseitig ist die Steuermembran (44) durch den Atemdruck beziehungsweise den Ausatemdruck beaufschlagt, steuerseitig liegt der Steuerdruck über den Steuerdruckausgang (52) und die Steuerleitung (41) und den Steuereingang (51) an. Optional und in diesem Beispiel gezeigt kann in dem Steuerausgang (52) eine Drossel (56) vorgesehen sein.

Die Steuermembrane (44) kann als PEEP-Membran ausgelegt beziehungsweise als solche funktionell realisiert sein, sodass der geringste Druckwert eines Atemzyklusses am Ende der Exspiration in der Lunge die Steuerbasis darstellt. Unter PEEP versteht man den "Positive End Expiratory Pressure".

Das als Umhausung der Steuermembran (44) ausgebildete Drucksteuerelement (40) weist einen Deckel (42) auf, der durch eine Schnappverbindung (43) gekoppelt ist und drehsicher ausgeführt wird, um die Montage zu vereinfachen und Fehlmontagen zu verhindern.

Das in Figur 3 gezeigte Ausführungsbeispiel weist in dem zweiten Ventilelement (20) eine Rückschlagmembran (22) auf, die eine Rückatmung in den Beatmungsschlauch beziehungsweise in das zweite Ventilelement (20) verhindert. Die Rückschlagmembran (22) ist axial derart stirnendseitig in dem zweiten Ventilelement (20) verortet, dass das sich bildende Totraumvolumen (VT) wenigstens reduziert ist. Dadurch lässt sich neben der für die Totraumreduzierung günstigen Längenreduzierung des Patientenventils (1) durch die geometrische Gestaltung des wenigstens einen Anschlusses (50) in abgewinkelter Form nochmals eine Totraumverringerung erzielen.

Um Fehlmontagen zu verhindern, ist die Verbindungsstelle (30) dieses Ausführungsbeispiels als verstecksicherer Konus (32) ausgebildet. Um die Kopplung der beiden Ventilelemente (10, 20) durchzuführen, wird im ersten Schritt die Verbindung an der Verbindungsstelle (30) durch axiales Ineinanderschieben hergestellt und im zweiten Schritt die Steuerleitung (41) mit den Anschlüssen (51, 52) verbunden.

## Patentansprüche

1. Patientenventil (1) zur Beatmung eines Patienten mit einem Beatmungsgerät, aufweisend ein erstes Ventilelement (10) und ein zweites Ventilelement (20), wobei das erste Ventilelement (10) wenigstens einen Anschluss (50) aufweist, wobei der wenigstens eine Anschluss (50) einen CO₂-Mess-Anschluss (53) und einen Atemwegsdruck-Messanschluss (54) umfasst, wobei der wenigstens eine Anschluss (50) mit seiner Mittenachse in einem zur Patientenventilmittenachse von der lotrechten Lage abweichenden Winkel ausgerichtet ist, sodass ein längenreduziertes Patientenventil (1) mit einem verminderten Totraumvolumen (VT) unterstützt ist, und wobei das erste Ventilelement (10) mit dem zweiten Ventilelement (20) an einer Verbindungsstelle (30) gekoppelt ist, und wobei das zweite Ventilelement (20) wenigstens eine Rückschlagmembran (22) aufweist, die axial derart stirnendseitig in dem zweiten Ventilelement verortet ist, sodass das sich bildende Totraumvolumen (VT) wenigstens reduziert ist, **dadurch gekennzeichnet, dass** das zweite Ventilelement (20) wenigstens einen Sauerstoffeinspeisungsanschluss (55) aufweist, sodass Sauerstoff in beliebig konzentrierter Form in das zweite Ventilelement (20) einbringbar ist.

2. Patientenventil (1) nach Anspruch 1, **dadurch gekennzeichnet, dass** der Winkel in einem Winkelbereich von ca. 25 Grad bis ca. 75 Grad zwischen Anschluss- und Patientenventilmittenachse realisiert ist.

3. Patientenventil (1) nach Anspruch 1, **dadurch gekennzeichnet, dass** der Winkel in einem Winkelbereich von ca. 30 Grad bis ca. 50 Grad zwischen Anschluss- und Patientenventilmittenachse realisiert ist.

4. Patientenventil (1) nach Anspruch 1, **dadurch gekennzeichnet, dass** die Anschlüsse (53, 55) in Längsrichtung des Patientenventils (1) fluchtend oder mit einem Winkel zueinander in Umfangsrichtung ausgerichtet sind.

5. Patientenventil (1) nach Anspruch 1, **dadurch gekennzeichnet, dass** das erste Ventilelement (10) ein Drucksteuerelement (40) aufweist.

6. Patientenventil (1) nach Anspruch 5, **dadurch gekennzeichnet, dass** das Drucksteuerelement (40) einen Deckel (42) aufweist, der durch eine Schnappverbindung (43) gekoppelt und verdrehsicher ausgeführt ist.

7. Patientenventil (1) nach Anspruch 5, **dadurch gekennzeichnet, dass** das Drucksteuerelement (40) eine Steuermembran (44) aufweist.

8. Patientenventil (1) nach Anspruch 7, **dadurch gekennzeichnet, dass** die Steuermembran (44) eine PEEP-Steuermembran ist.

9. Patientenventil (1) nach Anspruch 7, **dadurch gekennzeichnet, dass** die Steuermembran (44) über einen Steuereingangsanschluss (50, 51) druckbeaufschlagbar ist.

10. Patientenventil (1) nach Anspruch 1, **dadurch gekennzeichnet, dass** die Verbindungsstelle (30) eine Trennebene (31) aufweist.

11. Patientenventil (1) nach Anspruch 1, **dadurch gekennzeichnet, dass** die Verbindungsstelle (30) durch einen Konus (32) verstecksicher ausgebildet ist.

12. Patientenventil (1) nach Anspruch 9, 4- **dadurch gekennzeichnet, dass** das zweite Ventilelement (20) wenigstens einen Steueranschluss (50, 52) aufweist, sodass das Drucksteuerelement (40) an seinem Steuereingang (51) mit dem Steueranschluss (50, 52) wirkverbindbar und die Steuermembran (44) druckbeaufschlagbar ist.

13. Patientenventil (1) nach Anspruch 12, **dadurch gekennzeichnet, dass** eine Steuerleitung (41) die Wirkverbindung herstellt.

14. Patientenventil (1) nach Anspruch 1, **dadurch gekennzeichnet, dass** das Patientenventil als Einwegventil zur einmaligen Verwendung ausgebildet ist.

15. Patientenventil (1) nach Anspruch 1, **dadurch gekennzeichnet, dass** das Patientenventil (1) ein Ausatemventil ist.

## Claims

1. Patient valve (1) for ventilating a patient with a ventilator, comprising a first valve element (10) and a second valve element (20), wherein the first valve element (10) comprises at least one connector (50), wherein the at least one connector (50) comprises a CO2 measuring connection (53) and one airway pressure measuring port (54), where the at least one connector (50) is aligned with its central axis at an angle to the patient valve central axis that deviates from the vertical, such that a reduced-length patient valve (1) with a reduced dead space volume (VT) is supported, and wherein the first valve element (10) is coupled to the second valve element (20) at a connection point (30) and wherein the second valve element (20) comprises at least one non-return diaphragm (22), which is located axially at the end face of the second valve element in such a way that the resulting dead space volume (VT) is at least reduced, **characterized in that** the second valve element (20) has at least one oxygen feed connection (55), such that oxygen can be introduced into the second valve element (20) in any concentrated form.

2. Patient valve (1) according to claim 1, **characterized in that** the angle is realized in an angle range of approx. 25 degrees to approx. 75 degrees between the connection axis and the patient valve center axis.

3. Patient valve (1) according to claim 1, **characterized in that** the angle is realized in an angle range of approx. 30 degrees to approx. 50 degrees between the connection axis and the patient valve center axis.

4. Patient valve (1) according to claim 1, **characterized in that** the connections (53, 55) are aligned in the longitudinal direction of the patient valve (1) or at an angle to one another in the circumferential direction.

5. Patient valve (1) according to claim 1, **characterized in that** the first valve element (10) has a pressure control element (40).

6. Patient valve (1) according to claim 5, **characterized in that** the pressure control element (40) has a cover (42) which is coupled by a snap connection (43) and is designed to be torsion-proof.

7. Patient valve (1) according to claim 5, **characterized in that** the pressure control element (40) comprises a control diaphragm (44).

8. Patient valve (1) according to claim 7, **characterized in that** the control diaphragm (44) is a PEEP-control diaphragm.

9. Patient valve (1) according to claim 7, **characterized in that** the control diaphragm (44) can be pressurized via a control input connection (50, 51).

10. Patient valve (1) according to claim 1, **characterized in that** the connection point (30) has a separating plane (31).

11. Patient valve (1) according to claim 1, **characterized in that** the connection point (30) is made concealable by a cone (32).

12. Patient valve (1) according to claim 9 **characterized in that** the second valve element (20) has at least one control connection (50, 52), such that the pressure control element (40) can be operatively connected at its control input (51) to the control connection (50, 52) and the control diaphragm (44) can be pressurized.

13. Patient valve (1) according to claim 12, **characterized in that** a control line (41) establishes the active connection.

14. Patient valve (1) according to claim 1, **characterized in that** the patient valve is designed as a one-way valve for single use.

15. Patient valve (1) according to claim 1, **characterized in that** the patient valve (1) is an exhalation valve.

## Revendications

1. Valve de patient (1) destinée à ventiler un patient au moyen d'un ventilateur artificiel, ladite valve de patient comportant un premier élément de valve (10) et un deuxième élément de valve (20), le premier élément de valve (10) comportant au moins un raccord (50), l'au moins un raccord (50) comprenant un raccord de mesure de CO₂ (53) et un raccord de mesure de pression de voie respiratoire (54), l'au moins un raccord (50) étant orienté avec son axe central suivant un angle qui s'écarte de la position perpendiculaire par rapport à l'axe central de valve de patient, de sorte qu'une valve de patient (1) de longueur réduite est supportée avec un volume d'espace mort réduit (VT), et le premier élément de valve (10) étant accouplé au deuxième élément de valve (20) au niveau d'un point de liaison (30), et le deuxième élément de valve (20) comportant au moins une membrane anti-retour (22) qui est située axialement du côté de l'extrémité frontale du deuxième élément de valve de manière au moins à réduire le volume d'espace mort (VT) formé, **caractérisé en ce que** le deuxième élément de valve (20) comporte au moins un raccord d'alimentation en oxygène (55), de sorte que l'oxygène puisse être introduit dans le deuxième élément de valve (20) sous n'importe quelle forme concentrée.

2. Valve de patient (1) selon la revendication 1, **caractérisée en ce que** l'angle est formé dans une plage angulaire d'environ 25 degrés à environ 75 degrés entre l'axe central de raccord et l'axe central de valve de patient.

3. Valve de patient (1) selon la revendication 1, **caractérisée en ce que** l'angle est formé dans une plage angulaire d'environ 30 degrés à environ 50 degrés entre l'axe central de raccord et l'axe central de valve de patient.

4. Valve de patient (1) selon la revendication 1, **caractérisée en ce que** les raccords (53, 55) sont orientés en alignement dans la direction longitudinale de la valve de patient (1) ou suivant un angle l'un par rapport à l'autre dans la direction circonférentielle.

5. Valve de patient (1) selon la revendication 1, **caractérisée en ce que** le premier élément de valve (10) comporte un élément de commande de pression (40).

6. Valve de patient (1) selon la revendication 5, **caractérisée en ce que** l'élément de commande de pression (40) comporte un couvercle (42) qui est accouplé par une liaison par encliquetage (43) et qui est conçu pour être sécurisé en rotation.

7. Valve de patient (1) selon la revendication 5, **caractérisée en ce que** l'élément de commande de pression (40) comporte une membrane de commande (44).

8. Valve de patient (1) selon la revendication 7, **caractérisée en ce que** la membrane de commande (44) est une membrane de commande PEEP.

9. Valve de patient (1) selon la revendication 7, **caractérisée en ce que** la membrane de commande (44) peut être mise sous pression par le biais d'un raccord d'entrée de commande (50, 51).

10. Valve de patient (1) selon la revendication 1, **caractérisée en ce que** le point de liaison (30) présente un plan de séparation (31).

11. Valve de patient (1) selon la revendication 1, **caractérisée en ce que** le point de liaison (30) est conçu pour être masqué par un cône (32).

12. Valve de patient (1) selon la revendication 9, **caractérisée en ce que** le deuxième élément de valve (20) comporte au moins un raccord de commande (50, 52) de sorte que l'élément de commande (40) de pression puisse être relié fonctionnellement au raccord de commande (50, 52) au niveau de son entrée de commande (51) et que la membrane de commande (44) puisse être mise sous pression.

13. Valve de patient (1) selon la revendication 12, **caractérisée en ce qu'**une ligne de commande (41) établit la liaison fonctionnelle.

14. Valve de patient (1) selon la revendication 1, **caractérisée en ce que** la valve de patient est conçue comme une valve unidirectionnelle à usage unique.

15. Valve de patient (1) selon la revendication 1, **caractérisée en ce que** la valve de patient (1) est une valve d'expiration.
